# EUROPEAN PATENT SPECIFICATION

(11) **EP 2 361 377 B1**
(45) Date of publication and mention of the grant of the patent: **31.01.2018**
(21) Application number: 09748866.2
(22) Date of filing: 30.10.2009
(51) Int. Cl.: G01N 21/65, C12M 1/12, C12M 1/34, G01N 21/35, G01N 33/68, G01N 21/64, G01N 21/47, G01N 21/359, G01N 21/3581, C12Q 1/04

(54) **METHOD FOR IDENTIFICATION OF MICROORGANISMS USING RAMAN SPECTROSCOPY**
VERFAHREN ZUR IDENTIFIZIERUNG VON MIKROORGANISMEN MITTELS RAMAN-SPEKTROSKOPIE
PROCÉDÉ D'IDENTIFICATION DE MICROORGANISMES À L'AIDE DE LA SPECTROSCOPIE RAMAN

(30) Priority: 31.10.2008 US 110187 P
(43) Date of publication of application: 31.08.2011
(73) Proprietor: Biomerieux, Inc, Durham, NC 27712 (US)
(72) Inventor: CLAY, Bradford, Wildwood MO 63011 (US); HYMAN, Jones, Wake Forest NC 27587 (US); WALSH, John, Durham NC 27713 (US); THORPE, Thurman, Durham NC 27712 (US); RONSICK, Christopher, Durham NC 27717 (US)
(74) Representative: Murgitroyd & Company
(86) International application number: PCT/US2009/005886
(87) International publication number: WO 2010/062351

(56) References cited:
- WO-A2-2005/068647
- US-A- 4 212 948
- US-A1- 2002 086 289
- JARVIS ROGER M ET AL: "Surface-enhanced Raman scattering for the rapid discrimination of bacteria" FARADAY DISCUSSIONS, ROYAL SOCIETY OF CHEMISTRY, CAMBRIDGE, GB, vol. 132, 1 January 2006 (2006-01-01), pages 281-292;DISCU, XP009100498 ISSN: 1359-6640
- POPP J ET AL: "Raman-Spectroscopy for a rapid identification of single microorganisms" PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA, vol. 6180, 2005, pages 618024-1, XP002565157 ISSN: 0277-786X
- NEUGEBAUER U ET AL: "Characterization of bacterial growth and the influence of antibiotics by means of UV resonance Raman spectroscopy" BIOPOLYMERS WILEY USA, vol. 82, no. 4, July 2006 (2006-07), pages 306-311, XP002565156 ISSN: 0006-3525
- BERNHARDT M ET AL: "DETECTION OF BACTERIA IN BLOOD BY CENTRIFUGATION AND FILTRATION" JOURNAL OF CLINICAL MICROBIOLOGY, vol. 29, no. 3, 1991, pages 422-425, XP002565155 ISSN: 0095-1137
- MAQUELIN K ET AL: "Identification of medically relevant microorganisms by vibrational spectroscopy", JOURNAL OF MICROBIOLOGICAL METHODS, ELSEVIER, AMSTERDAM, NL, vol. 51, no. 3, 1 November 2002 (2002-11-01), pages 255-271, XP002479420, ISSN: 0167-7012, DOI: 10.1016/S0167-7012(02)00127-6

## Description

### CROSS REFERENCE TO RELATED APPLICATION

This application claims the benefit of U.S. Provisional Patent Application No. 61/110,187, entitled, "Method and System for Detection and/or Characterization of a Biological Particle in a Sample", filed October 31, 2008.

### FIELD OF THE INVENTION

The present invention relates to a method for identifying microorganisms in a sample. In particular, the present invention is directed to a method for the rapid identification of a microorganism using Raman spectroscopic techniques.

### BACKGROUND OF THE INVENTION

The detection of pathogenic microorganisms in biological fluids should be performed in the shortest possible time, in particular in the case of septicemia for which the mortality remains high in spite of the broad range of antibiotics which are available to doctors. The presence of biologically active agents such as a microorganism in a patient's body fluid, especially blood, is generally determined using blood culture bottles. Bloodstream infections are associated with high morbidity and mortality, yet current diagnostic methods, of culture followed by biochemical identification and antibiotic susceptibility testing, can take several days to perform. Typically, empiric therapy is initiated based on clinical symptoms, and test results only impact clinical decisions when the initial therapy fails. The ability to characterize bloodstream infections within the first few hours, preferably within an hour after a positive blood culture result would significantly boost the clinical relevance of the diagnostic information provided. Molecular amplification methods have been proposed to fill this need, but serious challenges to this approach remain. The positive blood culture broth itself represents a naturally amplified population of microorganisms with potential for use in a variety of rapid, identification (ID) tests.

Traditional automated phenotypic ID tests, such as the Vitek®, Phoenix™ and Microscan® systems, or manual phenotypic tests such as API require that microorganisms be in an appropriate growth phase and free of interfering media and blood products in order to provide robust results. These systems use colonies grown from the positive broth for 16-24 hours on plated media. However, in an effort to obtain faster results, some laboratories have reported using these systems with microorganisms isolated from positive blood culture bottles. These direct-from-the-bottle tests are not appropriate for all microorganisms (e.g., Gram-positive cocci), are not validated by the test manufacturers, and generally take 3-8 hours to provide results. Faster and more broadly specific tests are urgently needed in order to provide the physician with clinically relevant results within the first few hours, preferably within an hour after a positive culture result.

Raman spectroscopy has the potential to allow for identification of microorganisms very quickly, but may encounter interference from the many highly fluorescent and absorptive compounds present in liquid microbiological culture media and in clinical samples such as blood or combinations thereof. The most commonly employed methods for recovering microorganisms directly from positive blood culture broth are two-step differential centrifugation and centrifugation in a serum separator tube.

Other methods for identification of microorganisms have been described, include:

U.S. Pat. No. 4,847,198 discloses a method for the identification of microorganisms using UV excited Raman spectroscopy. According to the '198 patent, a bacterial suspension is contacted by a single wavelength in the ultra-violet range. A portion of the light energy used is absorbed and a portion of the light energy is emitted. The emitted light energy, resonance enhanced Raman scattering, is measured as backscattered energy. The energy is processed to produce spectra which are characteristic of the bacteria.

U.S. Pat. No. 5,938,617 to Vo-Dinh is directed to a system which identifies biological pathogens in a sample by exciting a sample with light at several wavelengths and synchronously sampling the emission intensities. The system includes mechanisms for exposing the sample to excitation radiation and thereby generating an emission radiation. The biological pathogens may be viruses and bacteria.

U.S. Pat. No. 6,177,266 discloses a method for the chemotaxonomic classification of bacteria with genus, species and strain specific biomarkers generated by matrix assisted laser desorption ionization time-of-flight mass spectrometry (MALDI-TOF-MS) analysis of either cellular protein extracts or whole cells.

In U.S. Pat. No. 7,070,739 a method is presented to extract, separate, and purify microbes including viruses by two-dimensional ultra-centrifuging directly from body fluids or homogenized tissue. In a first centrifuging step, all particles are removed having a sedimentation speed higher than those of the microbes to be identified. In the second ultra-centrifuging step, isopycnic banding is used in liquids filled in to form a wide-range density gradient, using special serrated centrifuge tubes. According to the patent, the separation technique can be used for detecting banded particles by light scatter or fluorescence using nucleic acid specific dyes, and for recovering the banded particles in very small volumes for characterization by mass spectrometry of viral protein subunits and intact viral particles, and by fluorescence flow cytometric determination of both nucleic acid mass and the masses of fragments produced by restriction enzymes.

U.S. Pat. Appl. Pub. No. 2007/0037135 discloses a system for the identification and quantification of a biological sample suspended in a liquid. The system includes a fluorescence excitation module with at least one excitation light source; a sample interface module optically coupled to the fluorescence excitation module for positioning a biological sample to receive excitation light from the at least one excitation light source; a fluorescence emission module optically coupled to the sample interface module and comprising at least one detection device for detecting fluorescence excitation-emission matrices of the biological sample; and a computer module operatively coupled to the fluorescence emission module. The computer module performs multivariate analysis on the fluorescence excitation-emission matrices of the biological sample to identify and quantify the biological sample. However, the '135 application does not discuss identification and quantification of microorganisms from complex biological samples, such as blood.

U.S. Pat. Appl. Pub. No. 2007/0175278 describes using a liquid culture medium for culturing a sample of interest, including for example, blood, urine, feces, intravenous catheters etc., industrial production lines, water systems, a food product, a cosmetic product, a pharmaceutical product and a forensic sample. Subsequently, the microorganisms can be harvested from the liquid medium by methods known in the art, e.g. by centrifugation. The concentrated microorganisms may then be transferred to carrier material, optionally after drying, for obtaining a vibrational spectrum. The patent application discusses various methods for identifying and classifying microorganisms, including vibrational spectroscopy, such as Raman spectroscopy.

However, these methods have several drawbacks when attempting to separate and characterize microorganisms from complex samples such as blood-containing culture media. The resultant microbial preparations often contain contaminating red blood cells, platelets, lipid particles, plasma enzymes and cellular debris, which can cause poor results. These methods are also very labor-intensive and unsafe due to steps which can result in aerosol exposure of potentially dangerous pathogens to the user. Simple, safe and reliable methods are needed to isolate microorganisms from clinical samples (e.g., blood culture broth) and other complex samples that are free of these interfering materials and compatible with rapid identification technologies. Maquelin et al "Identification of medically relevant microorganisms by vibrational spectroscopy", J. Microbiol. Meth., vol. 51, n. 3 (2002) discloses the identification of microorganisms from a test sample by Raman spectroscopy. US4 212 948 A discloses a method and means for detecting microbial pathogens.

### SUMMARY OF THE INVENTION

The present invention provides a method for identifying microorganisms in a sample. The method allows the identification of microorganisms more quickly than prior techniques, resulting in faster diagnoses (*e.g.,* in a subject having or suspected of having septicemia) and identification of contaminated materials (*e.g.,* foodstuffs and pharmaceuticals). The steps involved in the method of the invention, from obtaining a sample to identification of microorganisms, can be carried out in a very short time frame to produce clinically relevant actionable information, *e.g.,* in less than about 120 minutes. Additionally, the method of the invention can be fully automated, thereby reducing the risk of handling infectious materials and/or contaminating the samples.

The present invention is directed to a method of identifying a microorganism from a test sample according to claim 1.

In another aspect, the present invention is directed to a method of identifying a microorganism from a blood culture sample. The separation is carried out by layering the test sample over a density cushion in a container and centrifuging the container to pellet the microorganisms while the sample medium remains on top of the density cushion. In one example the container has an optical window at the bottom and/or sides so that the microorganism pellet can be interrogated spectroscopically. The microorganisms can be identified by comparing the spectrum of the pellet to a spectrum or spectra, or spectroscopic properties predicted, of known microorganisms. The ability to identify microorganisms directly in the pellet without further handling enhances the safety of microbial identification.

In one example the spectroscopic interrogation is based on interrogation of the vibrational structure of the constituent molecules that comprise the microorganisms. In other embodiments, the spectroscopic interrogation is based in part on signals obtained from additional agents that are added during the methods of the invention and interact with specific microorganisms or groups of microorganisms.

In another example the methods further comprise a step of recovering the microorganism pellet, resuspending the microorganism and performing further identification or characterization tests (*e.g.,* drug resistance, virulence factors, antibiogram).

The present invention is further defined in the claims.

The present invention is explained in greater detail in the figures herein and the description set forth below.

### BRIEF DESCRIPTION OF THE FIGURES

Figure 1 shows the Raman spectra of various microorganisms processed and recovered from blood culture.
Figure 2 shows the Raman spectra of 13 *S. aureus* isolates recovered directly from blood culture broth.
Figure 3 shows the Raman spectra read through a sealed separation device, with and without microorganisms present.
Figure 4 shows the Raman spectra of a blood culture-derived microorganism pellet read through the sealed separation device, background subtracted.

### DETAILED DESCRIPTION OF THE INVENTION

The present invention can be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

### Definitions.

As used herein, "a," "an," or "the" can mean one or more than one. For example, "a" cell can mean a single cell or a multiplicity of cells.

Also as used herein, "and/or" refers to and encompasses any and all possible combinations of one or more of the associated listed items, as well as the lack of combinations when interpreted in the alternative ("or").

Furthermore, the term "about," as used herein when referring to a measurable value such as an amount of a compound or agent of this invention, dose, time, temperature, and the like, is meant to encompass variations of ± 20%, ± 10%, ± 5%, ± 1%, ± 0.5%, or even ± 0.1% of the specified amount.

As used herein, the term "microorganism" is intended to encompass organisms that are generally unicellular, which can be multiplied and handled in the laboratory, including but not limited to, Gram-positive or Gram-negative bacteria, yeasts, molds, parasites, and mollicutes. Non-limiting examples of Gram-negative bacteria of this invention include bacteria of the following genera: *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Stenotrophomonas, Brevundimonas, Ralstonia, Achromobacter, Fusobacterium, Prevotella, Branhamella, Neisseria, Burkholderia, Citrobacter, Hafnia, Edwardsiella, Aeromonas, Moraxella, Brucella, Pasteurella, Providencia,* and *Legionella.* Non-limiting examples of Gram-positive bacteria of this invention include bacteria of the following genera: *Enterococcus, Streptococcus, Staphylococcus, Bacillus, Paenibacillus, Lactobacillus, Listeria, Peptostreptococcus, Propionibacterium, Clostridium, Bacteroides, Gardnerella, Kocuria, Lactococcus, Leuconostoc, Micrococcus, Mycobacteria* and *Corynebacteria.* Non-limiting examples of yeasts and molds of this invention include those of the following genera: *Candida, Cryptococcus, Nocardia, Penicillium, Alternaria, Rhodotorula, Aspergillus, Fusarium, Saccharomyces* and *Trichosporon.* Non-limiting examples of parasites of this invention include those of the following genera: *Trypanosoma, Babesia, Leishmania, Plasmodium, Wucheria, Brugia, Onchocerca,* and *Naegleria.* Non-limiting examples of mollicutes of this invention include those of the following genera: *Mycoplasma* and *Ureaplasma.*

In one embodiment, as described in further detail herein, microorganisms from a sample or growth medium can be separated and interrogated to characterize and/or identify the microorganism present in the sample. As used herein, the term "separate" is intended to encompass any sample of microorganisms that has been removed, concentrated or otherwise set apart from its original state, or from a growth or culture medium. For example, in accordance with this invention, microorganisms may be separated away (e.g., as a separated sample) from non-microorganism or non-microorganism components that may otherwise *i*nterfere with characterization and/or identification. The term may include a layer of microorganisms sandwiched between two other layers, *e.g.,* microorganisms collected on top of a high-density cushion after centrifugation, or a layer of microorganisms collected on a solid surface *(e.g.,* a filter membrane). The term may also include a collection of microorganisms that has passed partially through a layer (*e.g.,* a density cushion). As such, a separated microorganism sample may include any collection or layer of microorganisms and/or components thereof that is more concentrated than, or otherwise set apart from, the original sample, and can range from a closely packed dense clump of microorganisms to a diffuse layer of microorganisms. Microorganism components that can be comprised in a separated form or sample include, without limitation, pilli, flagella, fimbriae, and capsules in any combination. Non-microorganism components that are separated away from the microorganisms may include non-microorganism cells (e.g., blood cells and/or other tissue cells) and/or any components thereof.

As described in further detail herein, microorganisms from a sample or growth medium can be isolated and interrogated to identify the microorganism present in the sample. As used herein, the term "isolated" is intended to encompass any sample of microorganisms that has been at least partially purified from its original state, or from a growth or culture medium, and any non-microorganisms or non-microorganism components contained therein. For example, in accordance with this invention, microorganisms may be isolated away (e.g., as an isolated sample) from non-microorganisms or non-microorganism components that may otherwise interfere with characterization and/or identification. Non-microorganism components that are separated away from the microorganisms may include non-microorganism cells (e.g., blood cells and/or other tissue cells) and/or any components thereof.

As described in further detail herein, microorganisms from a sample or growth medium can be pelleted and interrogated to identify the microorganism present in the sample. As used herein, the term "pellet" is intended to encompass any sample of microorganisms that has been compressed or deposited into a mass of microorganisms. For example, microorganisms from a sample can be compressed or deposited into a mass at the bottom of a tube by centrifugation, or other known methods in the art. The term includes a collection of microorganisms (and/or components thereof) on the bottom and/or sides of a container following centrifugation. Microorganism components that can be comprised in a pellet include, without limitation, pilli, flagella, fimbriae, and capsules in any combination. In accordance with this invention, microorganisms may be pelleted away (e.g., as a substantially purified microorganism pellet) from non-microorganism or non-microorganism components that may otherwise interfere with characterization and/or identification. Non-microorganism components that are separated away from the microorganisms may include non-microorganism cells (e.g., blood cells and/or other tissue cells) and/or any components thereof.

As used herein, the term "density cushion" refers to a solution having a homogenous density throughout.

The method may be particularly useful for the separation, characterization and/or identification of microorganisms from complex samples such as blood-containing culture media. The rapid method also allow for the characterization and/or identification of microorganisms more quickly than prior techniques, resulting in faster diagnoses (*e.g.,* in a subject having or suspected of having septicemia) and characterization/identification of contaminated materials (*e.g.,* foodstuffs and pharmaceuticals). The steps involved in the method of the invention, from obtaining a sample to characterization/identification of microorganisms, can be carried out in a very short time frame to obtain clinically relevant actionable information. In certain embodiments, the methods of the invention can be carried out in less than about 120 minutes, *e.g.,* in less than about 110, 100, 90, 80, 70, 60, 50, 40, 30, 20, 15, 10, 5, 4, 3, 2, or 1 minute. The tremendous rapidity of the methods of the invention represents an improvement over prior methods. The method can be used to characterize and/or identify any microorganism as described herein. In one example the microorganism is a bacterium. In another example the microorganism is a yeast. In another embodiment, the microorganism is a mold. In a further embodiment, the microorganism is a parasite. In another example the microorganism is a mollicute. Additionally, the method of the invention can be fully automated, thereby reducing the risk of handling infectious materials and/or contaminating the samples.

In one aspect, the present invention is directed to a method of identifying a microorganism from a test sample.

In another aspect, the present invention is directed to a method of identifying a microorganism from a blood culture sample.

In another example of the invention, the method involve recovering the pellet of microorganisms formed during the separation step or a portion thereof from the separation container prior to interrogation of the microorganisms. For example, after formation of the pellet, the fluids can be aspirated way from the pellet and the pellet resuspended in a suitable medium (*e.g.,* a medium in which the microorganisms are viable). The resuspended microorganisms can be removed from the separation container. The microorganisms can then be interrogated identification, *e.g.,* in the suspension or after they have been repelleted. In other examples the resuspended microorganisms can be interrogated in the separation container, *e.g.,* in the suspension or after they have been repelleted. In a further example microorganisms recovered from the pellet can be used directly for further interrogation (*e.g.,* Raman spectroscopy) without being resuspended.

### Samples

Samples that may be tested (i.e., a test sample) by the methods of the invention include both clinical and non-clinical samples in which microorganism presence and/or growth is or may be suspected, as well as samples of materials that are routinely or occasionally tested for the presence of microorganisms. The amount of sample utilized may vary greatly due to the versatility and/or sensitivity of the method. Sample preparation can be carried out by any number of techniques known to those skilled in the art although one of the advantages of the present invention is that complex sample types, such as, *e.g.,* blood, bodily fluids, and/or other opaque substances, may be tested directly utilizing the system with little or no extensive pretreatment. In one embodiment, the sample is taken from a culture. In another embodiment, the sample is taken from a microbiological culture (e.g., a blood culture). In another embodiment, the sample is suspected of, or known to, contain microorganisms therein.

Clinical samples that may be tested include any type of sample typically tested in clinical or research laboratories, including, but not limited to, blood, serum, plasma, blood fractions, joint fluid, urine, semen, saliva, feces, cerebrospinal fluid, gastric contents, vaginal secretions, tissue homogenates, bone marrow aspirates, bone homogenates, sputum, aspirates, swabs and swab rinsates, other body fluids, and the like. In another embodiment, the clinical sample can be cultured, and a culture sample used.

The present invention finds use in research as well as veterinary and medical applications. Suitable subjects from which clinical samples can be obtained are generally mammalian subjects, but can be any animal. The term "mammal" as used herein includes, but is not limited to, humans, non-human primates, cattle, sheep, goats, pigs, horses, cats, dog, rabbits, rodents (*e.g.,* rats or mice), *etc.* Human subjects include neonates, infants, juveniles, adults and geriatric subjects. Subjects from which samples can be obtained include, without limitation, mammals, birds, reptiles, amphibians, and fish.

Non-clinical samples that may be tested also include substances, encompassing, but not limited to, foodstuffs, beverages, pharmaceuticals, cosmetics, water (*e.g.,* drinking water, non-potable water, and waste water), seawater ballasts, air, soil, sewage, plant material (*e.g.,* seeds, leaves, stems, roots, flowers, fruit), blood products (*e.g.,* platelets, serum, plasma, white blood cell fractions, *etc*.), donor organ or tissue samples, biowarfare samples, and the like. The method is also particularly well suited for real-time testing to monitor contamination levels, process control, quality control, and the like in industrial settings. In another embodiment, the non-clinical sample can be cultured, and a culture sample used.

In one example of the invention, samples are obtained from a subject (*e.g.,* a patient) having or suspected of having a microbial infection, for instance septicemia, *e.g.,* bacteremia or fungemia. The sample may be a blood sample directly from the subject. The sample may be from a blood culture grown from a sample of the patient's blood, *e.g.,* a BacT/ALERT® blood culture. The blood culture sample may be from a positive blood culture, *e.g.,* a blood culture that indicates the presence of a microorganism. In certain examples the sample is taken from a positive blood culture within a short time after it turns positive, *e.g.,* within about 6 hours, *e.g.,* within about 5, 4, 3, or 2 hours, or within about 60 minutes, *e.g.,* about 55, 50, 45, 40, 35, 30, 25, 20, 15, 10, 5, 4, 3, 2, or 1 minute. In one example the sample is taken from a culture in which the microorganisms are in log phase growth. In another embodiment, the sample is taken from a culture in which the microorganisms are in a stationary phase.

The present invention provides high sensitivity for the identification of microorganisms. This enables identification without first having to go through the steps of isolating microorganisms by growing them on a solid or semisolid medium, and sampling the colonies that grow. Thus, in one example of the invention, the sample is not from a microbial (*e.g.,* bacteria, yeast, or mold) colony grown on a solid or semisolid surface. Thus, in one example of the invention, the sample is not from a microbial (*e.g.,* bacteria, yeast, or mold) colony grown on a solid or semisolid surface.

The volume of the sample should be sufficiently large to produce an isolated sample of microorganisms or a pellet of microorganisms which can be interrogated after the separation/isolation step of the methods of the invention is carried out. Appropriate volumes will depend on the source of the sample and the anticipated level of microorganisms in the sample. For example, a positive blood culture will contain a higher level of microorganisms per volume than a drinking water sample to be tested for contamination, so a smaller volume of blood culture medium may be needed as compared to the drinking water sample. In general, the sample size can be less than about 50 ml, *e.g.,* less than about 40, 30, 20, 15, 10, 5, 4, 3, or 2 ml. The sample size can be about 1 ml, *e.g.,* about 0.75, 0.5, or 0.25 ml. In certain examples in which the separation is carried out on a microscale, the sample size can be less than about 200 µl, *e.g.,* less than about 150, 100, 50, 25, 20, 15, 10, or 5 µl. In some examples (*e.g.,* when the sample is expected to comprise a small number of microorganisms), the sample size can be about 100 ml or more, *e.g.,* about 250, 500, 750, or 1000 ml or more.

### Lysis Step

After obtaining a sample, the next step in the method of the present invention is to selectively lyse undesired cells that may be present in the sample, *e.g.,* blood cells and/or tissue cells. Cells may be lysed to permit separation of microorganisms from other components of the sample. The separation of microorganisms from other components prevents interference during the interrogation step. If non-microorganism cells are not expected to be present in the sample or not expected to interfere with the interrogation step, the lysis step may not need to be carried out. In one embodiment, the cells to be lysed are non-microorganism cells that are present in the sample and no microorganism cells that may be present in the sample are lysed. However, in some embodiments, the selective lysing of specific classes of microorganisms may be desirable and thus can be carried out according to the methods described herein and as are well known in the art. For example, a class of undesired microorganisms can be selectively lysed, *e.g.,* yeast are lysed while bacteria are not or vice versa. In another embodiment, the desired microorganisms are lysed in order to separate a particular subcellular component of the microorganisms, *e.g.,* cell membranes or organelles. In one embodiment, all of the non-microbial cells are lysed. In other embodiments, a portion of the non-microbial cells are lysed, *e.g.,* enough cells to prevent interference with the interrogation step. The lysing of cells may be carried out by any method known in the art to be effective to selectively lyse cells with or without lysing microorganisms, including, without limitation, addition of a lysis solution, sonication, osmotic shock, chemical treatment, and/or a combination thereof.

A lysis solution is one that is capable of lysing cells, *e.g.,* non-microorganism cells (*e.g.,* by solubilizing eukaryotic cell membranes) and/or microorganism cells. In one embodiment, the lysis solution can comprise one or more detergents, one or more enzymes, or a combination of one or more detergents and one or more enzymes, and can further include additional agents. In one embodiment, the detergent can be a non-denaturing lytic detergent, such as Triton® X-100 Triton® X-100-R, Triton® X-114, NP-40, Genapol® C-100, Genapol® X-100, Igepal® CA 630, Arlasolve™200, Brij® 96/97, CHAPS, octyl β-D-glucopyranoside, saponin, and nonaethylene glycol monododecyl ether (C12E9, polidocenol). Optionally, denaturing lytic detergents can be included, such as sodium dodecyl sulfate, N-laurylsarcosine, sodium deoxycholate, bile salts, hexadecyltrimethylammonium bromide, SB3-10, SB3-12, amidosulfobetaine-14, and C7BzO. Optionally, solubilizers can also be included, such as Brij® 98, Brij® 58, Brij® 35, Tween® 80, Tween® 20, Pluronic® L64, Pluronic® P84, non-detergent sulfobetaines (NDSB 201), amphipols (PMAL-C8), and methyl-β-cyclodextrin. Typically, non-denaturing detergents and solubilizers are used at concentrations above their critical micelle concentration (CMC), while denaturing detergents may be added at concentrations below their CMC. For example, non-denaturing lytic detergents can be used at a concentration of about 0.010% to about 10%, *e.g.,* about 0.015% to about 1.0%, *e.g.,* about 0.05% to about 0.5%, *e.g.,* about 0.10% to about 0.30% (final concentration after dilution with the sample). In another embodiment, polyoxyethylene detergent detergents may be preferred. The polyoxyethylene detergent can comprise the structure C₁₂₋₁₈/E₉₋₁₀, wherein C12-18 denotes a carbon chain length of from 12 to 18 carbon atoms and E9-10 denotes from 9 to 10 oxyethylene hydrophilic head groups.

Enzymes that can be used in lysis solutions include, without limitation, enzymes that digest nucleic acids and other membrane-fouling materials (*e.g.,* proteinase XXIII, DNase, neuraminidase, polysaccharidase, Glucanex®, and Pectinex®). Other additives that can be used include, without limitation, reducing agents such as 2-mercaptoethanol (2-Me) or dithiothreitol (DTT) and stabilizing agents such as magnesium, pyruvate, and humectants. The lysis solution can be buffered at any pH that is suitable to lyse the desired cells, and will depend on multiple factors, including without limitation, the type of sample, the cells to be lysed, and the detergent used. In some embodiments, the pH can be in a range from about 2 to about 13, *e.g.,* about 6 to about 13, *e.g.,* about 8 to about 13, *e.g.,* about 10 to about 13. Suitable pH buffers include any buffer capable of maintaining a pH in the desired range, *e.g.,* about 0.05 M to about 1.0 M CAPS.

In one example the sample and the lysis solution are mixed and then incubated for a sufficient time for lysis and solubilization of cell membranes to occur, *e.g.,* about 1, 2, 3, 4, 5, 10, 15, 20, 25, 30, 40, 50, or 60 seconds, or about 2, 3, 4, 5, 6, 7, 8, 9, 10, 15, or 20 minutes or longer, *e.g.,* about 1 second to about 20 minutes, about 1 second to about 5 minutes, or about 1 second to about 2 minutes. The incubation time will depend on the strength of the lysis solution, *e.g.,* the concentration of the detergent and/or enzymes. In general, milder lysis buffers will require more time and a greater dilution of the sample to fully solubilize non-microbial cells. The strength of the lysis solution can be selected based on the microorganisms known to be or suspected to be in the sample. For microorganisms that are more susceptible to lysis, a mild lysis solution can be used. The lysis can take place at a temperature of about 2°C to about 45°C, *e.g.,* about 15°C to about 40°C, *e.g.,* about 30°C to about 40°C. In one example the lysis solution can be loaded into a syringe and the sample can then be aspirated into the syringe such that mixing and incubation occurs within the syringe. In one embodiment, the lysis solution can be loaded into a syringe and the sample can then be aspirated into the syringe such that mixing and incubation occurs within the syringe.

In some examples the lysis conditions (*e.g.,* the solution or the incubation time), as well as the separation and/or interrogation steps, can be sufficient to kill some or all of the microorganisms in the sample. The method of the present invention is highly versatile and do not require that all microorganisms be alive for the isolation and identification to occur. In certain embodiments, some or all of the microorganisms may be dead, with death occurring before, during, and/or after the steps of the methods being carried out.

Further details and description of the lysis buffers contemplated in the practice of this invention are disclosed in pending U.S. patent application, serial no. _, filed October 30, 2009, entitled "Methods for Isolation and Identification of Microorganisms".

### Separation Step

The next step in the method of the present invention (e.g., the step after the sample has been lysed, if a lysing step is performed) is a separation step. The separation step can be carried out to separate the microorganisms from other components of the sample (e.g., non-microorganisms or components thereof) and to concentrate the microorganisms into a pellet that can be interrogated for identification and characterization purposes. The separation does not have to be complete, *i.e.,* it is not required that 100% separation occur. All that is required is that the separation of the microorganisms from other components of the sample be sufficient to permit interrogation of the microorganisms without substantial interference from the other components. For example, the separation can result in a microorganism pellet that is at least about 10, 20, 30, 40, 50, 60, 70, 80, 90, 95, 96, 97, 98, or 99% pure or higher.

In one example the separation is carried out by a centrifugation step in which the sample (*e.g.,* a lysed sample) is placed on top of a density cushion in a separation container and the container centrifuged under conditions which allow the microorganisms to be isolated (e.g., the microorganisms can form a pellet at the bottom and/or sides of the container). In accordance with this example other components of the sample (e.g., non-microorganisms or components thereof that may be present in the sample medium) stay on top of the density cushion or within the top portion of the density cushion. In general, any known container may be used for the separation step. In one example the separation container is the separation device disclosed in related U.S. patent application, serial no. _, filed October 30, 2009 entitled "Separation Device for Use in the Separation, Characterization and/or Identification of Microorganisms". This separation step isolates the microorganisms away from materials in the sample, such as medium, cell debris, and/or other components that might interfere with interrogation of the microorganisms (*e.g.,* by intrinsic fluorescence). In one example the density cushion also serves to separate live microorganisms from dead microorganisms (which do not pass through the density cushion). In another example the density cushion does not comprise a density gradient, either before or after the centrifugation. In other words, the separation container is not centrifuged for a sufficient amount of time and/or acceleration for the material making up the density cushion to form a density gradient.

The density of the cushion is selected such that the microorganisms in the sample pass through the cushion while other components of the sample (*e.g.,* blood culture broth, cell debris) remain on top of the cushion or do not pass all of the way through the density cushion. The density cushion may also be selected to separate live microorganisms (which pass through the cushion) from dead microorganisms (which do not pass through the cushion). Suitable densities will depend on the material used in the density cushion and on the sample to be separated. The density of the cushion is in the range of about 1.025 to about 1.120 g/ml, *e.g.,* about 1.030 to about 1.070 g/ml, about 1.040 to about 1.060 g/ml or any range between about 1.025 to about 1.120 g/ml. In another example the density of the cushion is about 1.025, 1.030, 1.035, 1.040, 1.045, 1.050, 1.055, 1.060, 1.065, 1.070, 1.075, 1.080, 1.085, 1.090, 1.095, 1.100, 1.105, 1.110, 1.115, or 1.120 g/ml.

The material for the density cushion can be any material that has the appropriate density range for the methods of this invention. In one embodiment, the material is colloidal silica. The colloidal silica may be uncoated (*e.g*., Ludox® (W.R. Grace, CT)) or coated, *e.g.,* with silane (*e.g.,* PureSperm® (Nidacon Int'l, Sweden) or Isolate® (Irvine Scientific, Santa Ana, CA)) or polyvinylpyrrolidone (*e.g*., Percoll™, Percoll™ Plus (Sigma-Aldrich, St. Louis, MO)). In one embodiment, the colloidal silica exhibiting the least interference with spectroscopic interrogation is selected, *e.g.,* the material with the lowest intrinsic fluorescence. The colloidal silica may be diluted in any suitable medium to form the proper density, *e.g.,* balanced salt solutions, physiological saline, and/or 0.25 M sucrose. Suitable densities can be obtained with colloidal silica at a concentration of about 15% to about 80% v/v, *e.g.,* about 20% to about 65% v/v. Another suitable material for density cushions is an iodinated contrast agent, *e.g.,* iohexol (Omnipaque™ NycoPrep™, or Nycodenz®) and iodixanol (Visipaque™ or OptiPrep™). Suitable densities can be obtained with iohexol or iodixanol at a concentration of about 10% to about 25% w/v, *e.g.,* about 14% to about 18% w/v, for blood culture samples. Sucrose can be used as a density cushion at a concentration of about 10% to about 30% w/v *e.g.,* about 15% to about 20% w/v, for blood culture samples. Other suitable materials that can be used to prepare the density cushion include low viscosity, high density oils, such as microscope immersion oil (*e.g.,* Type DF; Cargille Labs, New York), mineral oil (*e.g*., Drakeol® 5, Draketex 50, Peneteck®; Penreco Co., Pennsylvania), silicone oil (polydimethylsiloxane), fluorosilicone oil, silicone gel, metrizoate-Ficoll® (LymphoPrep™), *e.g.,* at a concentration of about 75% to about 100% for blood culture samples, diatrizoate-dextran (PolymorphoPrep™), *e.g.,* at a concentration of about 25% to about 50% for blood culture samples, carboxymethyl cellulose, hydroxypropylmethyl cellulose, polyethylene oxide (high molecular weight), Pluronic® F127, Pluronic® F68, mixtures of Pluronic® compounds, polyacrylic acid, cross-linked polyvinyl alcohol, cross-linked polyvinyl pyrrolidine, PEG methyl ether methacrylate, pectin, agarose, xanthan, gellan, Phytagel®, sorbitol, Ficoll® (*e.g*., Ficoll® 400 at a concentration of about 10% to about 15% for blood culture samples), glycerol, dextran (*e.g.,* at a concentration of about 10% to about 15% for blood culture samples), glycogen, cesium chloride (*e.g.,* at a concentration of about 15% to about 25% for blood culture samples), perfluorocarbon fluids (*e.g.,* perfluoro-n-octane), hydrofluorocarbon fluids (*e.g.,* Vertrel XF), and the like as are well known in the art. In one embodiment, the density cushion is selected from one or more of colloidal silica, iodixanol, iohexol, cesium chloride, metrizoate-Ficoll®, diatrizoate-dextran, sucrose, Ficoll® 400, and/or dextran in any combination. The density cushion can also be made up of a combination of materials, *e.g.,* a combination of colloidal silica and oil. Certain combinations of the above compounds may be beneficial for the separation and reading steps of the present invention. For example, combinations of compounds with different UV-quenching properties, such as cesium chloride and Iohexol.

The volume/height of the density cushion should be sufficient to achieve separation of the microorganisms from other sample components. The volume will depend on the size and shape of the separation container. In general, a volume of about 0.1 to about 5 ml can be used, *e.g.,* about 0.2 to about 1 ml, *e.g.,* about 0.2 ml to about 0.5 ml. If the separation is performed on a microscale, the volume of the density cushion can be about 1 µl to about 100 µl, *e.g.,* about 5 µl to about 50 µl. The volume of sample laid or layered on top of the density cushion should be sufficient to provide enough microorganisms to produce a pellet suitable for interrogation. In general, any volume that fits into the container can be used. For example, a volume of about 0.1 ml to about 5 ml can be used, *e.g.,* about 0.2 ml to about 1 ml, *e.g.,* about 0.2 ml to about 0.5 ml. If the separation is performed on a microscale, the volume of sample can be about 1 µl to about 100 µl, *e.g.,* about 5 µl to about 50 µl. The available space in the container for sample will depend on the size and shape of the container. In some embodiments, an intermediate layer (liquid or solid) can be placed on top of the density cushion before the sample is laid or layered on top in order to prevent any mixing of the density cushion and the sample. In one embodiment, the intermediate layer can be polyethylene beads. In another embodiment, a small air bubble can be positioned between the density cushion and the sample to prevent mixing. In a further embodiment, the density cushion can be layered on top of a high density material (*e.g.,* a perfluorocarbon fluid) such that the microorganisms pass through the density cushion during the separation and collect at the interface between the density cushion and the high density material.

In one example of the invention, the separation container is centrifuged in a swing out rotor so that the microorganisms form a pellet directly on the bottom of the container. The container is centrifuged at a sufficient acceleration and for a sufficient time for the microorganisms to be separated (e.g., a pellet formed) from other components of the sample. The centrifugation acceleration can be about 1,000 x *g* to about 20,000 x *g, e.g.,* about 2,500 x *g* to about 15,000 x *g, e.g.,* about 7,500 x *g* to about 12,500 x *g, etc.* The centrifugation time can be about 30 seconds to about 30 minutes, *e.g.,* about 1 minute to about 15 minutes, *e.g.,* about 1 minute to about 5 minutes. The centrifugation can be carried out at a temperature of about 2°C to about 45°C, *e.g.,* about 15°C to about 40°C, *e.g.,* about 20°C to about 30°C. In one example the separation container comprises a closure, and the closure is applied to the container to form a hermetic seal prior to centrifugation. The presence of a closure decreases the risks from handling microorganisms that are or may be infectious and/or hazardous, as well as the risk of contaminating the sample. One of the advantages of the methods of the invention is the ability to carry out any one or more of the steps of the methods (*e.g.,* lysis, separation, interrogation, and/or identification) with the microorganisms in a sealed container (*e.g.,* a hermetically sealed container). The present methods, involving the use of automated systems, avoid the health and safety risks associated with handling of highly virulent microorganisms, such as occurs with recovery of microorganisms from samples for direct testing. In one embodiment, the container is not centrifuged for a sufficient time and/or force for a density gradient to form within the density cushion. The present invention does not involve ultracentrifugation of samples, *e.g.,* centrifugation at forces greater than about 100,000 x *g.* Further, the present invention does not involve isopycnic (equilibrium) sedimentation or banding.

The separation container may be any container with sufficient volume to hold a density cushion and a sample. As noted herein, the separation device disclosed in related U.S. patent application, serial no. _, filed October 30, 2009, entitled "Separation Device for Use in the Separation, Characterization and/or Identification of Microorganisms", may be used in the practice of this invention. In one embodiment, the container fits or can be fitted into a centrifuge rotor. The volume of the container can be about 0.1 ml to about 25 ml, *e.g.,* about 1 ml to about 10 ml, *e.g.,* about 2 ml to about 8 ml. If the separation is done on a microscale, the volume of the container can be about 2 µl to about 100 µl, *e.g.,* about 5 µl to about 50 µl. In one embodiment, the container has a wide internal diameter in an upper portion to hold the sample and the majority of the density cushion, and a more narrow internal diameter in a lower portion where the pellet of microorganisms is collected. The narrow portion can have an internal diameter of about 0.04 to about 0.12 inches, *e.g.,* about 0.06 to about 0.10 inches, *e.g.,* about 0.08 inches. The wide portion can have an internal diameter of about 0.32 to about 0.40 inches, *e.g.,* about 0.34 to about 0.38 inches, *e.g.,* about 0.36 inches. For microscale separations, the internal diameters can be even smaller. For example, the internal diameter of the narrow portion can be about 0.001 to about 0.04 inches, *e.g.,* about 0.002 to about 0.01 inches. A tapered internal diameter portion can connect the upper and lower portions. The tapered portion can have an angle of about 20 to about 70 degrees, *e.g.,* about 30 to about 60 degrees. In one embodiment, the lower narrow portion is less than half of the total height of the container, *e.g.,* less than about 40%, 30%, 20%, or 10% of the total height of the container. The container can have a closure device attached or may be threaded to accept a closure device (*e.g.,* a cap) such that the container can be hermetically sealed during centrifugation. In certain embodiments, the container is designed such that the microorganism sample or pellet can be readily recovered, or otherwise obtained or removed from the container after separation, either manually or in an automated manner (so that technicians are not exposed to the container contents). For example, the container can comprise a removable portion or a break-away portion which contains the pellet and which can be separated from the rest of the container. In another embodiment, the container comprises means for access to the pellet after separation, such as one or more ports or permeable surfaces for insertion of a syringe or other sampling device or for drawing off the pellet. In one embodiment, the container can be a tube, *e.g.,* a centrifuge tube. In another embodiment, the container can be a chip or a card. In one embodiment, the container is a stand alone container, *i.e.,* a device for separating a single sample. In other embodiments, the container is part of a device that comprises two or more separation containers such that multiple samples can be separated at the same time. In one embodiment, the device comprises 2, 3, 4, 5, 6, 7, 8, 9, 10, 12, 15, 20, 25, 30, 36, 42, 48, 60, 72, 84, 96, or more separation containers.

The container can comprise an optical window through which the interrogation can occur. The optical window may be on the bottom, top, and/or sides of the container. The window can be comprised of any material that has a vibrational structure which is distinguishable from the spectra of the microorganism. Other discrimination techniques such as confocal Raman spectroscopy can be utilized to acquire the vibrational spectra of the microorganism while rejecting the spectra of the window material; this technique is well-known to those skilled in the art. An additional technique is Spatially Offset Raman Spectroscopy in which the excitation fiber is displaced along the window from the emission (Rayleigh and Raman spectra). This technique is also known to those skilled in the art as a means of discriminating between a window material and a quantity to be measured beneath the window. To enable interrogation of the microbial pellet with additional spectroscopic techniques, the window can also be composed of any material that is transparent to light (*e.g.,* at least a portion of the near infrared (NIR; 700 nm-1400 nm), ultraviolet (UV; 190 nm-400 nm) and/or visible (VIS; 400 nm-700 nm) light spectrum). In one embodiment, the optical window is thin enough to permit spectroscopic interrogation, which will depend on the material of the window and the method used for interrogation. In another embodiment, the optical window is as thin as possible to reduce interference with spectroscopic interrogation. For example, the window can have a thickness of less than about 0.20 inches, *e.g.,* less than about 0.15, 0.10, or 0.05 inches.

In another example the separation is carried out by a filtration step in which the sample (*e.g.,* a lysed sample) is placed in a device fitted with a selective filter or filter set with pore sizes that retain the microorganisms. The retained microorganisms may be washed by gently passing a suitable buffer through the filter. The washed microorganisms may then be interrogated directly on the filter and/or recovered for interrogation by directly sampling the surface of the filter or by back-flushing the filter with suitable aqueous buffer.

### Interrogation Step

Once the microorganisms have been separated, isolated and/or pelleted, the separated sample, isolated sample or pellet can be interrogated to identify the microorganisms in the sample or pellet. The interrogation takes place in a non-invasive manner, that is, the pellet is interrogated while it remains in the separation container. In another embodiment, the separation container remains sealed throughout the interrogation. The ability to identify the microorganisms in a non-invasive manner, optionally coupled with keeping the container sealed throughout the separation and identification/characterization process and automating some or all of the procedure avoids the constant handling of contaminated and/or infectious samples and greatly increases the safety of the entire process. Furthermore, the ability to identify microorganisms by direct interrogation without further processing of the pellet (*e.g.,* resuspension, plating, and growth of colonies), greatly increases the speed with which identification can be made. In one embodiment, the pellet is recovered and/or resuspended and optionally removed from the separation container prior to interrogation. In another embodiment, the pellet is recovered and/or resuspended after *in situ* interrogation and further interrogation is then carried out. For example, techniques such as latex agglutination tests or automated phenotypic identification tests that can be applied to isolated microorganisms but not a pellet of microorganisms can be carried out on the recovered and/or resuspended microorganisms.

The isolated sample or pellet is interrogated spectroscopically. The spectroscopy can be used to analyze one or more intrinsic properties of the microorganisms, *e.g.,* a property present within the microorganism in the absence of additional agents, such as stains, dyes, binding agents, *etc.* In other embodiments, the spectroscopy and be used to analyze one or more extrinsic properties of the microorganisms, *e.g.,* a property that can only be detected with the aid of additional agents. The interrogation can be carried using. Raman spectroscopy, including Surface Enhanced Raman Spectroscopy (SERS), Spatially Offset Raman spectroscopy (SORS), transmission Raman spectroscopy, and/or resonance Raman spectroscopy. To enhance Raman (SERS) signals, microorganisms could either be coated with gold and/or silver nanoparticles prior to centrifugation, and/or the inner optical surface could be precoated with metal colloids of particular size and shape (Efrima et al., J. Phys. Chem. B. (Letter) 102:5947 (1998) for SERS). In another example the nanoparticles are present in the density cushion prior to centrifugation and associate with microorganisms as the microorganisms pass through the density cushion. The isolated sample or pellet is interrogated while it remains in the separation container. The container can be interrogated through an optical window in the container. The optical window may be on the bottom and/or any side or sides and/or on the top of the container. In one embodiment, the separation container fits into or can be fitted into a holder in a spectrometer in a suitable position for interrogation. The spectroscopic interrogation can be carried out by any technique known to those of skill in the art to be effective for identifying one or more intrinsic or extrinsic properties of microorganisms.

The sample excitation source may be selected from any number of suitable light sources as known to those skilled in the art. Any portion of the electromagnetic spectrum that produces usable data can be used. Light sources capable of emission in the ultraviolet, visible and/or near-infrared spectra, as well as other portions of the electromagnetic spectrum, can be utilized and are known to those skilled in the art. Since the generation of Raman spectra is generally an inefficient process (e.g. 1 Raman photon generated for every 1 million incident photons), Raman requires a light source with a large photon flux. Since the invention of the laser in 1960 (Maiman, Nature), Raman systems have used a laser light source for obtaining Raman spectra. The laser light is passed through a highly selective (notch) filter that allows the narrow wavelength band of laser emission to transmit through the filter while blocking other artifacts generated by the laser (e.g. amplified spontaneous emission, fluorescence, plasma lines, etc.)

The light from the laser is guided to the sample using a variety of optical systems which can be created using relay lens systems, optical fibers, or a combination of both. The laser light is focused onto the sample using a lens (such as a microscope objective) so that a small spot of concentrated optical energy at the sample is obtained.

Raman backscattered light and backscattered excitation light are collected and collimated by the same microscope objective. The scattered light traverses through an optical filter that separates the Raman scattered light from the excitation light. The Raman scattered light is then propagated using a lens and/or optical fiber system to a spectrometer which disperses the Raman scattered light so that it impinges onto a CCD detector array.

The spatial dispersion of the Raman scattered light generates a spectra (e.g. scattered wavelength versus intensity) which can be stored an compared to reference spectra for characterization.

The emission from the sample may be measured by any suitable means of spectral discrimination, most preferably employing a spectrometer. The spectrometer may be a scanning monochromator that detects specific emission wavelengths whereby the output from the monochromator is detected by a photomultiplier tube and/or the spectrometer may be configured as an imaging spectrograph whereby the output is detected by an imaging detector array such as a charge-coupled device (CCD) detector array. In one embodiment, a discriminator allows the observation of the fluorescence and/or scattering signal by a photodetection means (such as a photomultiplier tube, avalanche photodiode, CCD detector array, and/or electron multiplying charge coupled device (EMCCD) detector array).

According to the invention, control measurements are taken for known microorganisms, thus allowing for correlation of measured test data with characterization of the microorganisms of interest using various mathematical methods known to those skilled in the art. For example, the data from samples may be compared with the baseline or control measurements utilizing software systems known to one skilled in the art. More particularly, the data may be analyzed by a number of multivariate analysis methods, such as, for example, General Discriminant Analysis (GDA), Partial Least Squares Discriminant Analysis (PLSDA), Partial Least Squares regression, Principal Component Analysis (PCA), Parallel Factor Analysis (PARAFAC), Neural Network Analysis (NNA) and/or Support Vector Machine (SVM). These methods may be used to classify unknown microorganisms of interest into relevant groups based on existing nomenclature, and/or into naturally occurring groups based on the organism's metabolism, pathogenicity and/or virulence in designing the system for monitoring, detecting and/or characterizing the organism as described previously.

Non-spectroscopic measurements from the detection system, such as detection times and growth rates can be used to assist in the identification of microorganisms from the isolated sample or pellet. Additionally, measurements taken from a photographic image of the lower region of the separation device can provide valuable information on the identity of the isolate, such as pellet size, shape, color and density.

In some examples of the invention, identification of the microorganisms in the isolated sample or pellet need not involve identification of an exact species. Characterization encompasses the broad categorization or classification of biological particles as well as the actual identification of a single species. Classification of microorganism from an isolated sample or pellet may comprise determination of phenotypic and/or morphologic characteristics for the microorganism. For example, characterization of the biological particles may be accomplished based on observable differences, such as, composition, shape, size, clustering and/or metabolism. In some embodiments, classification of the biological particles of interest may require no prior knowledge of the characteristics of a given biological particle but only requires consistent correlations with empiric measurements thus making this method more general and readily adaptable than methods based on specific binding events or metabolic reactions. As used herein "identification" means determining to which family, genus, species, and/or strain a previously unknown microorganism belongs to. For example, identifying a previously unknown microorganism to the family, genus, species, and/or strain level.

In some instances, characterization encompasses classification models which provide sufficient useful information for action to be taken. As used herein, the preferred classification models comprise grouping into one or more of the following: (1) Gram Groups; (2) Clinical Gram Groups; (3) Therapeutic Groups; and (4) Functional Groups..
(1) Gram Groups: Within the Gram Groups classification, microorganisms may be placed into one of three broad classification categories based on their Gram staining reaction and overall size, said groups selected from one or more of the following: (a) Gram positive microorganisms that stain dark blue with Gram staining; (b) Gram negative microorganisms that stain red with Gram staining; and (c) yeast cells that stain dark blue with Gram staining, but are very large rounded cells that are distinguished from bacteria by their morphological characteristics and size.
(2) Clinical Gram Groups: The Gram Groups may be further divided into several sub-categories representing distinguishing morphological features. These sub-categories comprise all the relevant clinical information reported by an experienced laboratory technologist, and thus provide a higher level of identification than a positive or negative Gram reaction. This particular classification is very helpful because it eliminates concerns about relying on the quality of a Gram stain and/or the skill level of the technician reading the smear by providing the equivalent clinically relevant information with an automated system. More specifically, subcategories of microorganisms based on this classification model may be selected from one or more of the following: (a) cocci, which are small rounded cells; (b) diplococci, which are two small rounded cells joined together; (c) rods, which are rectangular shape; and (d) bacilli, which are rod shaped. Examples of these sub-categories that can be ascertained by additional morphological information include: (i) Gram positive cocci; (ii) Gram positive cocci in chains; (iii) Gram positive cocci in clusters (*i.e.,* "grape-like" clusters); (iv) Gram positive diplococci; (v) Gram positive rods; (vi) Gram positive rods with endospores; (vii) Gram negative rods; (viii) Gram negative coccobacilli; (ix) Gram negative diplococci; (x) yeast; and (xi) filamentous fungi.
(3) Therapeutic Groups: The therapeutic groups comprise multiple microbial species that, when isolated from particular specimen types, are treated with the same class of antibiotics or mixture of antibiotics (*e.g.,* as described in *"*Sanford Guide to Antimicrobial Therapy 2008"). In many cases, identity to the species level is not required by the clinician to enable a change from initial empiric therapy to a more targeted therapy because more than one species can be treated with the same choice of antibiotic(s). This classification level correctly places these "same-treatment" microorganisms into single therapeutic categories. Examples of this characterization level include the ability to distinguish highly resistant Enterobacteriacae (EB) species from sensitive EB species (*Enterobacter* spp. from *E. coli*), or fluconazole-resistant *Candida* species (*C. glabrata* and *C. kruzei*) from sensitive *Candida* species (*C. albicans* and *C. parapsilosis*), and so on.
(4) Functional Groups: According to the invention, microorganisms may also be placed into several groups based upon a mixture of metabolic, virulence and/or phenotypic characteristics. Non-fermentative organisms may be clearly distinguished from fermentative ones. Furthermore, microorganism species that produce hemolysins may be grouped separately from non-hemolytic species. In some cases, these groups represent broader categories than genus level (*e.g*., coliforms, Gram negative non-fermentative rods), some at the genus level (*e.g., Enterococcus, Candida*), and some with closer to species-level discrimination (*e.g.*, coagulase-negative staphylococci, alpha-hemolytic streptococci, beta-hemolytic streptococci, coagulase-positive staphylococci, *i.e., S. aureus*).

In addition to measuring intrinsic properties of microorganisms for identification purposes, the method can further comprise the use of additional identifier agents to aid in the separation and/or identification process. Agents that bind to specific microorganisms, such as affinity ligands, can be used to separate microorganisms, to identify a class or species of microorganism (*e.g.,* through binding to a unique surface protein or receptor) and/or to identify a characteristic of the microorganism (*e.g.,* antibiotic resistance). Useful identifier agents include, without limitation, monoclonal and polyclonal antibodies and fragments thereof *(e.g.,* anti-Eap for *S. aureus* identification), nucleic acid probes, antibiotics (*e.g.,* penicillin, vancomycin, polymyxin B), aptamers, peptide mimetics, phage-derived binding proteins, lectins, host innate immunity biomarkers (acute phase proteins, LPS-binding protein, CD14, mannose binding lectin, Toll-like receptors), host defense peptides (*e.g.,* defensins, cathelicidins, proteogrins, magainins), bacterocins (*e.g.,* lantibiotics, such as nisin, mersacidin, epidermin, gallidermin, and plantaricin C, and class II peptides), bacteriophages, and dyes selective for nucleic acids, lipids, carbohydrates, polysaccharides, capsules/slime or proteins, or any combination thereof. If the agent does not itself give out a detectable Raman signal, the agent can be labeled to provide a detectable signal, such as by conjugating the agent to a Raman active tracer, such as dimethylaminoazobenzene (DAB) and 4,4"-dipyridyl (DPY) (see, e.g., U.S. Pat. Nos. 5,376,556 and 7,518,721). The agent can be added to the microorganisms at any step in the methods of the invention, *e.g.,* when the sample is obtained, during lysis, and/or during separation. In some examples the presence of the agent in the pellet can be determined during interrogation of the pellet. As would be readily appreciated by one of skill in the art, the sensitivity of a particular microorganism to any compound affecting its physical state or metabolism, such as an antibiotic, could be rapidly ascertained by adding the compound to the sample, lysis buffer, density cushion or any mixture thereof.

The method can further comprise a step of recovering the pellet of microorganisms and performing additional tests. In one embodiment, the pellet can be recovered by aspirating off the sample medium and density cushion. In another example the pellet can be recovered by inserting a syringe into the container and aspirating out the pellet while the sample medium and density cushion remain intact. The recovered pellet can then be resuspended in a suitable medium, *e.g.,* saline. Once resuspended, the microorganisms can be subject to any further tests that are desired, as would be known to those of skill in the art and as described above. In particular, any test requiring clean samples of microorganisms can be carried out with the resuspended microorganisms. Additional identification tests can be performed. Examples of identification tests include Vitek® 2, amplified and non-amplified nucleic acid tests (NAT), chromogenic and latex agglutination assays, immunoassays, (*e.g.,* employing labeled antibodies and/or other ligands), mass spectrometry (*e.g.,* MALDI-TOF mass spectrometry) and/or other optical techniques such as infrared spectroscopy (FTIR) or Raman spectroscopy. Additional characterization tests can also be performed, such as resistance to antibiotics and/or other drugs. The additional characterization may be part of a test that was started during the initial separation and identification steps of the method. For example, the detection of methicillin resistant *S. aureus* can begin by adding Raman tracer-labeled penicillin to the sample prior to separation of the microorganisms. Once the pellet has been recovered and resuspended, the level of bound penicillin can be determined.

Some or all of the method steps can be automated. Automating the steps of the method not only allows more samples to be tested more quickly, it also reduces the risks of human errors in handling samples that may contain harmful and/or infectious microorganisms.

In one example the pellet is detected with the naked eye.

In some examples the detection method can be used to monitor samples for contamination by microorganisms, *e.g.,* foodstuffs, pharmaceuticals, drinking water, *etc.* In one embodiment, the methods can be carried out in a repetitive fashion for constant monitoring for contamination, *e.g.,* once a month, once a week, once a day, once an hour, or any other time pattern. In another example samples can be tested as needed, *e.g.,* when contamination is suspected. In further examples the detection method can be used to look for the presence of microorganisms in clinical samples, *e.g.,* blood cultures. For example, a sample can be removed from a blood culture at certain time points and the detection method carried out on the sample to determine if the blood culture is positive. In one example a sample may be taken at a set time point after inoculation of the culture, *e.g.,* 24 hours after inoculation, to determine if the blood culture is positive. In another example samples can be taken from the blood culture regularly, *e.g.,* every 12, 6, 4, or 2 hours or every 60, 50, 40, 30, 20, 15, 10, or 5 minutes, to identify positive blood cultures within a short time of being detectably positive.

Some or all of the method steps can be automated. Automating the steps of the method allows a greater number of samples to be tested more efficiently and reduces the risks of human errors in handling samples that may contain harmful and/or infectious microorganisms. Of greater importance, however, automation can deliver critical results at any time of the day or night without delay. Several studies have shown that faster identification of the organisms causing sepsis correlates with improved patient care, shorter hospital stays and lower overall costs.

Further details and description of the method for identifying microorganisms, in accordance with the present invention, are disclosed in pending U.S. patent applications, serial nos. _ and _, both filed October 30, 2009, entitled "Method for Separation, Characterization and/or Identification of Microorganisms using Spectroscopy" and "Method for Separation, Characterization and/or Identification of Microorganisms using Mass Spectrometry".

The present invention is further detailed in the following examples, which are offered by way of illustration and is not intended to limit the invention in any manner. Standard techniques well known in the art or the techniques specifically described below are utilized.

### EXAMPLES

### Example 1. Lysis-Centrifugation Method for Identification of Microorganisms from Blood Cultures by Raman Spectroscopy.

Microorganisms were "seeded" at a low inoculum into BacT/ALERT® SA bottles containing 10 mLs of human blood. Blood culture broth samples were removed from bottles within a few minutes of being flagged positive by the BacT/ALERT® 3D Microbial Detection System. Broth samples were processed to separate microorganisms from blood and media components that could interfere with subsequent analysis as follows:

4.0 mL of broth from freshly positive blood cultures was combined with 2.0 mL Lysis Buffer (0.45% Brij® 97 in 0.3M CAPS, pH 11.7), vortex mixed for 5 seconds and then incubated in a 37°C waterbath for 90 seconds. After incubation, 0.95 mL of lysate was layered on top of 0.5 mL of density cushion (14% w/v Iohexol, 0.005% Pluronic F-108 in 10 mM Hepes, pH 7.4) in each of four 1.5 mL conical centrifuge tubes. All four tubes were then centrifuged for 2 minutes at 10,000 g at 25°C to sediment (pellet) the microorganisms through the density cushion. The lysed blood and media remained above the cushion.

Upon completion of the centrifuge cycle, the supernatant was removed and the sedimented (pelleted) microorganisms in each tube were resuspended with 10 µL of purified water. The resuspended microorganisms from all 4 tubes were pooled into a clean tube and mixed gently. The volume of each processed specimen was then adjusted so that the optical density at 660 nm (A₆₆₀) of the final suspension was equal to 20/cm. The processed specimens were either stored at 2-8°C for same-day testing, or aliquoted and frozen at -70°C for testing on a later date.

### Example 2. Analysis of microorganism specimens processed from positive blood cultures with Lysis-Centrifugation by Raman Spectroscopy.

Specimens processed according to the procedure in Example 1 were rapidly thawed at 37°C (if previously frozen), mixed gently. A portion of the specimen was kept undiluted and the remainder was diluted to 1:2 in purified water. One microliter of each diluted and undiluted specimen was applied to the first surface of a gold-coated microscope slide and allowed to dry under ambient conditions.

After drying, 25 spectra in a 5x5 grid pattern were acquired for each of the dried specimens with a Model RxN1 Raman microscope (Kaiser Optical Systems Inc., Michigan) at an illumination wavelength of 785 nm. The microscope was equipped with a 40X objective lens, the laser power was set to 400 mw, and each of the 25 individual spectra was acquired with a 5 second accumulation time.

After acquisition, the spectra were preprocessed to perform dark subtraction, cosmic ray artifact removal, spectral truncation, baseline subtraction, normalization, and outlier removal in preparation for and multivariate statistical analysis.

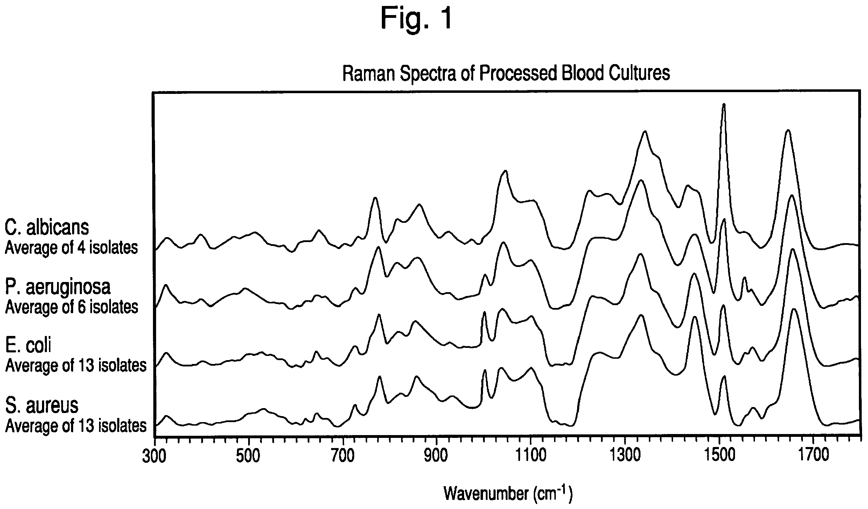

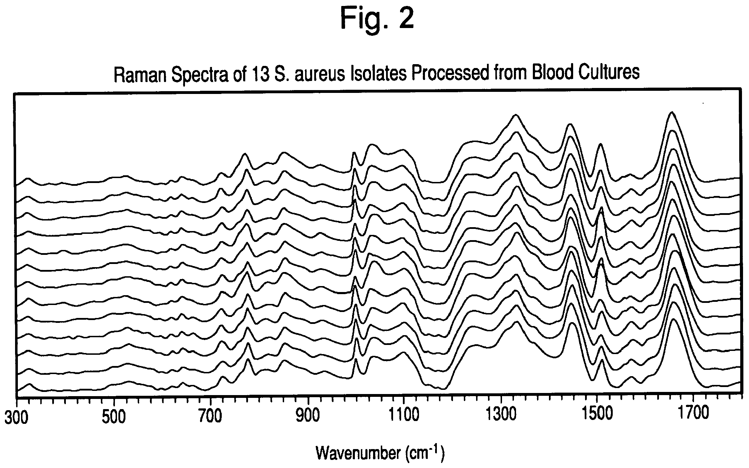

Representative Raman spectra of selected microorganisms recovered and processed from positive blood cultures are shown in Figures 1 and 2. Figure 1 shows spectra of four species of microorganisms; four isolates of *C. albicans,* six isolates of *P. aeruginosa,* thirteen isolates of *E. coli,* and thirteen isolates of *S. aureus.* Spectra from each species were averaged for clarity of illustration.

The consistent differences across the spectra of different species are readily apparent for these organisms. This shows that potentially obscuring blood and culture media have been removed sufficiently to obtain characteristic spectra for the microorganisms after processing.

Figure 2 shows the 13 individual Raman spectra of the *S.aureus* isolates that were shown averaged in Figure 1. The thirteen spectra show the consistency of the Raman spectrum for this microorganism across different clinical isolates, even when grown in different blood cultures with different blood donors.

### Example 3. Non-invasive analysis of microorganism specimens processed from positive blood cultures with Lysis-Centrifugation by Raman Spectroscopy.

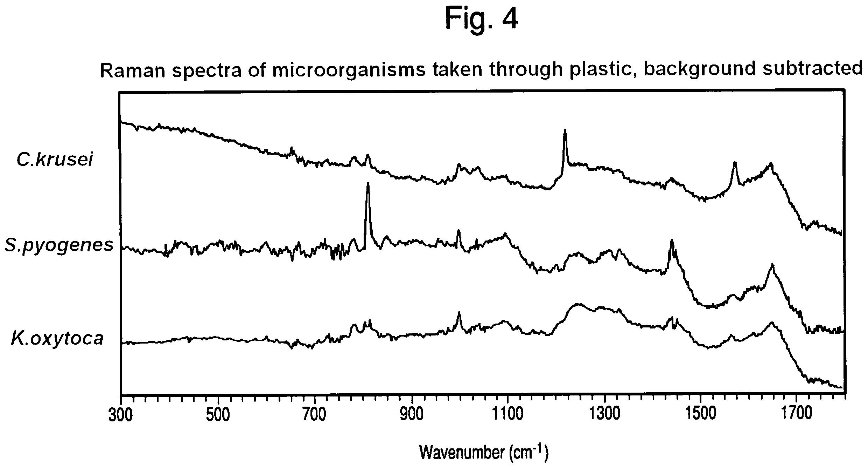

Microorganisms were "seeded" at a low inoculum into BacT/ALERT® SA bottles containing 10 mL of human blood. Blood culture broth samples were removed from bottles within a few minutes of being flagged positive by the BacT/ALERT® 3D Microbial Detection System. The samples were treated as follows:
1. A 2.0 mL sample of positive broth was mixed with 1.0 mL of selective lysis buffer (0.45% w/v Brij® 97 + 0.3M CAPS, pH 11.7), then placed in a 37°C water bath for 1 minute.
2. A 1.0 mL sample of lysate was overlayed onto 0.5 mL of density cushion (24% w/v cesium chloride in 10 mM Hepes ph 7.4 + 0.005% Pluronic F-108) contained in a custom-built optical separation tube. A polypropylene ball was present on the surface of the density cushion to facilitate loading without disturbing the two aqueous phases.
3. The optical separation tube was sealed with a screw-cap and centrifuged for 2 minutes at 10,000 rpm (Eppendorf® 5417R micrcentrifuge fitted with a A-8-11 swing out rotor; see Figure 4).
4. The sealed tube was transferred to a custom-built adapter which facilitated interrogation of the microbial pellet in the base of the tube by a Model RxN1 Raman microscope (Kaiser Optical Systems Inc., Michigan) at an illumination wavelength of 785 nm. Spectra were also recorded of empty separation tubes to establish baseline Raman spectra of the plastic.

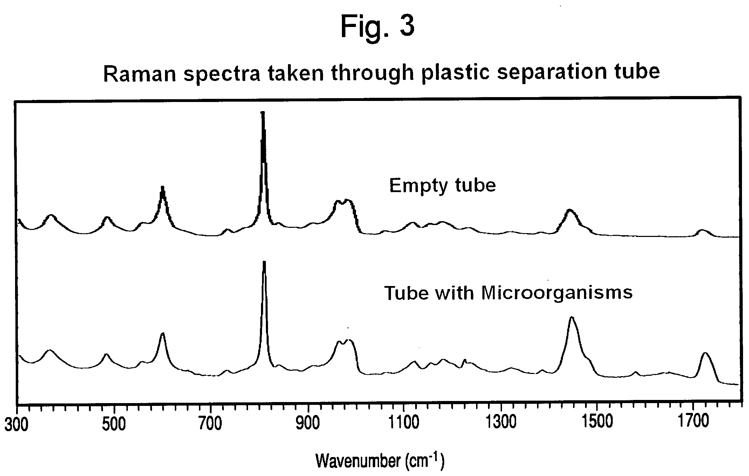

Figure 3 shows the Raman spectra taken through the plastic separation tube both with and without a microorganism pellet. In this mode, the spectra are dominated by Raman bands from the plastic itself; however, bands unique to the microorganisms are visible only in the spectrum of the microbial pellet at wavenumbers near 1227/cm, 1583/cm, and 1660/cm. Other microorganism bands are also present, but are obscured by the stronger plastic bands at this scale.

Figure 4 shows the Raman spectra of three microorganisms after the background has been removed by subtracting the spectrum of the empty plastic separation tube. While this background subtraction is not 100% effective, the spectral bands of the microorganisms are clearly visible. Other acquisition geometries, such as spatially offset or transmission acquisition, are likely to improve the background compensation further (see, e.g., U.S. patent appl. publ. nos. 2008/0129992 and 2009/0244533).

The foregoing Examples are illustrative of the present invention, and are not to be construed as limiting thereof. The invention is defined by the following claims.

## Claims

1. A method of identifying a microorganism from a test sample, comprising:
(a) obtaining a test sample known to contain or that may contain microorganisms;
(b) selectively lysing and solubilizing non-microorganism cells in said test sample with a lysis solution to produce a lysed sample, said lysis solution having a pH from 8 to 13;
(c) layering said lysed sample on a density cushion in a sealed container and centrifuging the container to separate said microorganisms from other components of said test sample and form a pellet of microorganisms, said microorganism passing through said density cushion to form a microorganism pellet at the bottom of said container;
(d) interrogating said pellet of microorganisms using one or more Raman spectroscopy techniques while said pellet remains in the container to produce spectroscopic measurements of said microorganism; and
(e) identifying said microorganism in said isolated sample to the family level, genus level, species level, and/or strain level by comparison of the spectroscopic measurements with spectroscopic measurements taken, or spectroscopic properties predicted, of known microorganisms.

2. The method of claim 1, wherein the density cushion has a density of from 1.025 to 1.120 g/ml.

3. The method of any preceding claim wherein steps (c), and (d) are carried out in a sealed container and wherein said interrogation step (d) is non-invasive.

4. The method of any preceding claim wherein said Raman spectroscopy technique is selected from the group consisting of Raman spectroscopy, confocal Raman spectroscopy, surface enhanced Raman spectroscopy, spatially offset Raman spectroscopy, resonance Raman spectroscopy, transmission Raman spectroscopy, and any combination thereof.

5. The method of any preceding claim wherein said microorganisms are characterized based on one or more phenotypic and/or morphologic characteristics.

6. The method of any preceding claim wherein said microorganisms are characterized based on one or more measurements of detection time, growth rate and microorganism pellet size, shape, color and/or density.

7. The method of any preceding claim wherein said microorganisms are characterized into on one or more classification models selected from the group consisting of Gram Groups, Clinical Gram Groups, Therapeutic Groups, and Functional Groups.

8. The method of any preceding claim wherein said lysis solution comprises one or more detergents preferably wherein said one or more detergents is Triton® X-100, Triton® X-100-R, Triton® X-114, NP-40, Genapol® C-100, Genapol® X-100, Igepal® CA 630, Arlasolve™200, Brij® 96/97, CHAPS, octyl β-D-glucopyranoside, saponin, nonaethylene glycol monododecyl ether (C12E9, polidocenol), sodium dodecyl sulfate, N-laurylsarcosine, sodium deoxycholate, bile salts, hexadecyltrimethylammonium bromide, SB3-10, SB3-12, amidosulfobetaine-14, C7BzO, Brij® 98, Brij® 58, Brij® 35, Tween® 80, Tween® 20, Pluronic® L64, Pluronic® P84, non-detergent sulfobetaines (NDSB 201), amphipols (PMAL-C8), methyl-β-cyclodextrin, or a polyoxyethylene detergent comprising the structure C₁₂₋₁₈/E₉₋₁₀.

9. The method of claim 8, wherein said lysis solution further comprises one or more enzymes, and wherein said one or more enzymes comprises a mixture of one or more proteinases and one or more nucleases and/or one or more buffering agents.

10. The method of any preceding claim wherein said density cushion is selected from the group consisting of colloidal silica, iodinated contrast agents, sucrose, microscope immersion oil, mineral oil, silicone oil, fluorosilicone oil, silicone gel, metrizoate-Ficoll®, diatrizoate-dextran, carboxymethyl cellulose, hydroxypropylmethyl cellulose, polyethylene oxide (high molecular weight), Pluronic® F127, Pluronic® F68, polyacrylic acid, cross-linked polyvinyl alcohol, cross-linked polyvinyl pyrrolidine, PEG methyl ether methacrylate, pectin, agarose, xanthan, gellan, Phytagel®, sorbitol, Ficoll®, glycerol, dextran, glycogen, cesium chloride, perfluorocarbon fluids, hydrofluorocarbon fluid, and combinations thereof.

11. The method of any preceding claim wherein said test sample is a culture sample known to contain microorganisms.

12. The method of claim 1, wherein the container remains sealed throughout the interrogation.

13. The method of claim 12, wherein said culture sample is a blood culture sample.

## Patentansprüche

1. Ein Verfahren zum Identifizieren eines Mikroorganismus aus einer Testprobe, beinhaltend:
(a) Erhalten einer Testprobe, die bekanntermaßen Mikroorganismen enthält oder diese enthalten kann;
(b) selektives Lysieren und Auflösen von Nicht-Mikroorganismus-Zellen in der Testprobe mit einer Lyselösung, um eine lysierte Probe zu erzeugen, wobei die Lyselösung einen pH-Wert von 8 bis 13 aufweist;
(c) Schichten der lysierten Probe auf ein Dichtekissen in einem abgedichteten Behälter und Zentrifugieren des Behälters, um die Mikroorganismen von anderen Komponenten der Testprobe zu trennen und ein Pellet aus Mikroorganismen zu bilden, wobei der Mikroorganismus durch das Dichtekissen geht, um am Boden des Behälters ein Mikroorganismus-Pellet zu bilden;
(d) Untersuchen des Pellets aus Mikroorganismen unter Verwendung einer oder mehrerer Raman-Spektroskopietechniken, während das Pellet in dem Behälter verbleibt, um spektroskopische Messungen des Mikroorganismus zu erzeugen; und
(e) Identifizieren des Mikroorganismus in der isolierten Probe auf Familienebene, Genusebene, Artenebene und/oder Stammebene durch Vergleich der spektroskopischen Messungen mit vorgenommenen spektroskopischen Messungen oder vorhergesagten spektroskopischen Eigenschaften bekannter Mikroorganismen.

2. Verfahren gemäß Anspruch 1, wobei das Dichtekissen eine Dichte von 1,025 bis 1,120 g/ml aufweist.

3. Verfahren gemäß einem der vorhergehenden Ansprüche, wobei die Schritte (c) und (d) in einem abgedichteten Behälter ausgeführt werden und wobei der Untersuchungsschritt (d) nichtinvasiv ist.

4. Verfahren gemäß einem der vorhergehenden Ansprüche, wobei die Raman-Spektroskopietechnik ausgewählt ist aus der Gruppe, bestehend aus Raman-Spektroskopie, konfokaler Raman-Spektroskopie, oberflächenverstärkter Raman-Spektroskopie, räumlich versetzter Raman-Spektroskopie, Resonanz-Raman-Spektroskopie, Transmissions-Raman-Spektroskopie und einer beliebigen Kombination davon.

5. Verfahren gemäß einem der vorhergehenden Ansprüche, wobei die Mikroorganismen auf der Basis eines oder mehrerer phänotypischer und/oder morphologischer Charakteristiken charakterisiert werden.

6. Verfahren gemäß einem der vorhergehenden Ansprüche, wobei die Mikroorganismen auf der Basis einer oder mehrerer Messungen von Erfassungszeit, Wachstumsrate und Größe, Form, Farbe und/oder Dichte der Mikroorganismen-Pellets charakterisiert werden.

7. Verfahren gemäß einem der vorhergehenden Ansprüche, wobei die Mikroorganismen in ein oder mehrere Klassifizierungsmodelle, ausgewählt aus der Gruppe, bestehend aus Gram-Gruppen, klinischen Gram-Gruppen, therapeutischen Gruppen und funktionellen Gruppen, charakterisiert werden.

8. Verfahren gemäß einem der vorhergehenden Ansprüche, wobei die Lyselösung ein oder mehrere Detergenzien beinhaltet, wobei vorzugweise das eine oder die mehreren Detergenzien Folgendes ist/sind: Triton® X-100, Triton® X-100-R, Triton® X-114, NP-40, Genapol® C-100, Genapol® X-100, Igepal® CA 630, Arlasolve™200, Brij® 96/97, CHAPS, Octyl-β-D-glucopyranosid, Saponin, Nonaethylenglycolmonododecylether (C12E9, Polidocenol), Natriumdodecylsulfat, N-Laurylsarcosin, Natriumdeoxycholat, Gallensalze, Hexadecyltrimethylammoniumbromid, SB3-10, SB3-12, Amidosulfobetain-14, C7BzO, Brij® 98, Brij® 58, Brij® 35, Tween® 80, Tween® 20, Pluronic® L64, Pluronic® P84, Nicht-Detergens-Sulfobetaine (NDSB 201), Amphipole (PMAL-C8), Methyl-βcyclodextrin oder ein Polyoxyethylendetergens, beinhaltend die Struktur C₁₂₋₁₈/E₉₋₁₀.

9. Verfahren gemäß Anspruch 8, wobei die Lyselösung ferner ein oder mehrere Enzyme beinhaltet und wobei das eine oder die mehreren Enzyme eine Mischung von einer oder mehreren Proteinasen und einer oder mehreren Nukleasen und/oder einem oder mehreren Puffermitteln beinhalten.

10. Verfahren gemäß einem der vorhergehenden Ansprüche, wobei das Dichtekissen ausgewählt ist aus der Gruppe, bestehend aus kolloidalem Siliciumdioxid, iodierten Kontrastmitteln, Saccharose, Mikroskopimmersionsöl, Mineralöl, Siliconöl, Fluorsiliconöl, Silicongel, Metrizoat-Ficoll®, Diatrizoat-Dextran, Carboxymethylcellulose, Hydroxypropylmethylcellulose, Polyethylenoxid (hochmolekular), Pluronic® F127, Pluronic® F68, Polyacrylsäure, vernetztem Polyvinylalkohol, vernetztem Polyvinylpyrrolidin, PEG-Methylethermethacrylat, Pectin, Agarose, Xanthan, Gellan, Phytagel®, Sorbit, Ficoll®, Glycerin, Dextran, Glycogen, Cäsiumchlorid, Perfluorcarbonfluiden, Hydrofluorkohlenstofffluid und Kombinationen davon.

11. Verfahren gemäß einem der vorhergehenden Ansprüche, wobei die Testprobe eine Kulturprobe ist, die bekanntermaßen Mikroorganismen enthält.

12. Verfahren gemäß Anspruch 1, wobei der Behälter während der gesamten Untersuchung abgedichtet bleibt.

13. Verfahren gemäß Anspruch 12, wobei die Kulturprobe eine Blutkulturprobe ist.

## Revendications

1. Une méthode d'identification d'un micro-organisme à partir d'un échantillon à tester, comprenant :
(a) l'obtention d'un échantillon à tester connu pour contenir ou qui est susceptible de contenir des micro-organismes ;
(b) la lyse et la solubilisation sélectives de cellules non micro-organismes dans ledit échantillon à tester avec une solution de lyse afin de produire un échantillon lysé, ladite solution de lyse ayant un pH allant de 8 à 13 ;
(c) la disposition en couche dudit échantillon lysé sur un coussin de densité dans un récipient fermé hermétiquement et la centrifugation du récipient afin de séparer lesdits micro-organismes d'autres constituants dudit échantillon à tester et former un culot de micro-organismes, ledit micro-organisme passant à travers ledit coussin de densité afin de former un culot de micro-organismes au fond dudit récipient ;
(d) l'interrogation dudit culot de micro-organismes en utilisant une ou plusieurs techniques de spectroscopie Raman tandis que ledit culot reste dans le récipient afin de produire des mesures spectroscopiques dudit micro-organisme ; et
(e) l'identification dudit micro-organisme dans ledit échantillon isolé au niveau de la famille, au niveau du genre, au niveau de l'espèce, et/ou au niveau de la souche par comparaison des mesures spectroscopiques avec des mesures spectroscopiques prises, ou des propriétés spectroscopiques prédites, de micro-organismes connus.

2. La méthode de la revendication 1, dans laquelle le coussin de densité a une densité allant de 1,025 à 1,120 g/ml.

3. La méthode de n'importe quelle revendication précédente dans laquelle les étapes (c) et (d) sont effectuées dans un récipient fermé hermétiquement et dans laquelle ladite étape d'interrogation (d) est non invasive.

4. La méthode de n'importe quelle revendication précédente dans laquelle ladite technique de spectroscopie Raman est sélectionnée dans le groupe constitué de la spectroscopie Raman, de la spectroscopie Raman confocale, de la spectroscopie Raman exaltée de surface, de la spectroscopie Raman spatialement décalée, de la spectroscopie Raman de résonance, de la spectroscopie Raman de transmission, et de toute combinaison de celles-ci.

5. La méthode de n'importe quelle revendication précédente dans laquelle lesdits micro-organismes sont caractérisés sur la base d'une ou de plusieurs caractéristiques phénotypiques et/ou morphologiques.

6. La méthode de n'importe quelle revendication précédente dans laquelle lesdits micro-organismes sont caractérisés sur la base d'une ou de plusieurs mesures de temps de détection, de taux de croissance, et de taille, forme, couleur et/ou densité de culot de micro-organismes.

7. La méthode de n'importe quelle revendication précédente dans laquelle lesdits micro-organismes sont caractérisés en un ou plusieurs modèles de classification sélectionnés dans le groupe constitué de Groupes Gram, de Groupes Gram Cliniques, de Groupes Thérapeutiques, et de Groupes Fonctionnels.

8. La méthode de n'importe quelle revendication précédente dans laquelle ladite solution de lyse comprend un ou plusieurs détergents préférablement dans laquelle lesdits un ou plusieurs détergents sont le Triton® X-100, le Triton® X-100-R, le Triton® X-114, le NP-40, le Genapol® C-100, le Genapol® X-100, l'Igepal® CA 630, l'Arlasolve™200, le Brij® 96/97, le CHAPS, l'octyl-β-D-glucopyranoside, la saponine, l'éther monododécylique de nonaéthylène glycol (C12E9, polidocénol), le dodécylsulfate de sodium, la N-laurylsarcosine, le désoxycholate de sodium, des sels biliaires, le bromure d'hexadécyltriméthylammonium, le SB3-10, le SB3-12, l'amidosulfobétaïne-14, le C7BzO, le Brij® 98, le Brij® 58, le Brij® 35, le Tween® 80, le Tween® 20, le Pluronic® L64, le Pluronic® P84, des sulfobétaïnes non détergentes (NDSB 201), des amphipols (PMAL-C8), la méthyl-β-cyclodextrine, ou un détergent polyoxyéthylène comprenant la structure C₁₂₋₁₈/E₉₋₁₀.

9. La méthode de la revendication 8, dans laquelle ladite solution de lyse comprend en sus une ou plusieurs enzymes, et dans laquelle lesdites une ou plusieurs enzymes comprennent un mélange d'une ou de plusieurs protéinases et d'une ou de plusieurs nucléases et/ou d'un ou de plusieurs agents tampons.

10. La méthode de n'importe quelle revendication précédente dans laquelle ledit coussin de densité est sélectionné dans le groupe constitué de silice colloïdale, d'agents de contraste iodés, de saccharose, d'huile à immersion pour microscope, d'huile minérale, d'huile de silicone, d'huile de fluorosilicone, de gel de silicone, de métrizoate-Ficoll®, de diatrizoate-dextrane, de carboxyméthyl cellulose, d'hydroxypropylméthyl cellulose, d'oxyde de polyéthylène (masse moléculaire élevée), de Pluronic® F127, de Pluronic® F68, d'acide polyacrylique, d'alcool polyvinylique réticulé, de polyvinyl pyrrolidine réticulée, de méthacrylate d'éther méthylique de PEG, de pectine, d'agarose, de xanthane, de gellane, de Phytagel®, de sorbitol, de Ficoll®, de glycérol, de dextrane, de glycogène, de chlorure de césium, de fluides perfluorocarbonés, de fluide hydrofluorocarboné, et de combinaisons de ceux-ci.

11. La méthode de n'importe quelle revendication précédente dans laquelle ledit échantillon à tester est un échantillon de culture connu pour contenir des micro-organismes.

12. La méthode de la revendication 1, dans laquelle le récipient reste fermé hermétiquement pendant toute l'interrogation.

13. La méthode de la revendication 12, dans laquelle ledit échantillon de culture est un échantillon d'hémoculture.
